# EUROPEAN PATENT SPECIFICATION

(11) **EP 1 981 481 B1**
(45) Date of publication and mention of the grant of the patent: **16.09.2015**
(21) Application number: 07700011.5
(22) Date of filing: 03.01.2007
(51) Int. Cl.: A61K 9/127, A61K 9/107, A61K 9/16, A61K 9/19, A61K 9/51

(54) **ORAL, PULMONARY AND TRANSMUCOSAL DELIVERY COMPOSITION**
ZUSAMMENSETZUNG ZUR ORALEN, PULMONALEN UND TRANSMUKOSALEN ABGABE
COMPOSITION DE DÉLIVRANCE ORALE, PULMONAIRE ET TRANSMUCOSALE

(30) Priority: 05.01.2006 US 756359 P; 27.12.2006 US 616658
(43) Date of publication of application: 22.10.2008
(73) Proprietor: Sköld, Thomas, 761 41 Norrtälje (SE)
(72) Inventor: Sköld, Thomas, 761 41 Norrtälje (SE)
(74) Representative: Sanderson, Nigel Paul
(86) International application number: PCT/IB2007/000027
(87) International publication number: WO 2007/077529

(56) References cited:
- EP-A1- 0 526 666
- WO-A-2005/092298

## Description

Provided are compositions and methods for oral, pulmonary and transmucosal delivery of bioactive agents.

US Patent Application 2005/129722 describes a foamy, viscous composition set forth in the Table found in Example 3 of this patent publication. The composition is said to be good for transdermally administering an active substance. The composition is made from a vesicle fraction, a foam fraction, and a hydrophilic fraction, and contains more than 8%, possibly more than 10%, by weight in lipid components. While abstractly the application recites any number of variables that might be varied, no teaching indicates that this thick formulation can be effectively diluted to a composition suitable for spraying, and which is nonetheless effective for transmucosal delivery of bioactive agents. In fact, diluting formulations like those described in this publication leads to the formation of unacceptable sediments.

The present application concerns oral, pulmonary and transmucosal delivery compositions that contain aqueous mixtures of three types of lipids: (1) phospholipids or certain similar lipids, (2) fatty acids, and (3) bilayer-stabilizing steroids. The variety of lipid structures that these lipids may form are believed to facilitate transport across mucosal membranes or epithelial tissue of intestines. In certain embodiments, the lipid structures are controlled so that the compositions contain one or both of two types of lipid aggregates, bilayer-enclosed vesicles and lipid particles. In many cases, the viscosity of the composition is selected to allow application by spraying, such as intranasal spraying. In some embodiments, the transmucosal delivery composition includes a conjugate of a lipid-phase anchoring hydrophobic moiety and a flexible, soluble polymer. Such conjugates have been thought to be contraindicated for transdermal compositions.

In certain embodiments, the present delivery compositions have properties that aid in avoiding side effects such as irritation to mucosal tissue. For example, the compositions can be formulated with low, non-irritating amounts of damaging cell-surface disruptors, such as organic solvents and strong detergents (such as Polysorbate 80). In certain embodiments, the compositions are essentially lacking in such cell-surface disruptors. In certain embodiments, the delivery compositions have lipid compositions sufficiently like the lipid composition of mucous membrane that, should any mucous membrane disruption occur in the delivery process, the compositions facilitate healing of such disruption.

Without being bound by theory, it is believed that by contacting mucosal cell membranes with mucosal-membrane-like lipid compositions, a system with enhanced entropy at the membrane bilayers is temporarily and reversibly formed. The enhanced entropy can facilitate trans-membrane transit, such as by enhancing active transport mechanisms, enhancing membrane fusion events that carry bioactive agent, by creating short-term holes or disorganized patches in the bilayers, or the like. Such energizing of the cell membranes is believed to be much more benign and short-lived than occurs with cell-surface disruptors. After such short-term energy enhancement, the delivery composition provides materials that are much like cell membrane, and which can be expected to be incorporated into cell membrane as part of a relatively rapid healing process.

### Summary of the Invention

Provided, in one embodiment, is a delivery composition comprising: an aqueous carrier; a lipid component suspended in the carrier comprising significant amounts of type A lipid, fatty acid, and bilayer-stabilizing steroid(s), wherein type A lipid is one or more of any of phospholipid, ceramide(s), sphingomyelin(s), glucocerebroside(s) and conjugate(s) of lipid-phase anchoring hydrophobic moieties and flexible, soluble polymers; and a bioactive agent, wherein (a) the delivery composition is packaged with a label with directions for mucosal, pulmonary or oral administration, and/or (b)(i) the viscosity of the composition is adjusted to a viscosity appropriate for spraying and/or (ii) the type A lipid comprises conjugate(s) of lipid-phase anchoring hydrophobic moieties and flexible, soluble polymers, and/or (iii) comprises a stabilizing effective amount of soluble polymers. The lipid component can comprise a lipid particle component; and optionally, a vesicle component comprising vesicles enclosed by substantially a single lipid bilayer.

Further provided is a method of treating a disease, disorder or condition comprising administering a delivery composition to a mucosal, lung or intestinal surface of a subject in need of a bioactive agent.

In certain embodiments, the delivery composition is formed by separately making (i) a lipid particle component and (ii) a vesicle component, then mixing the two components. The bioactive agent delivery profile (i.e., pharmacokinetic profile) can be adjusted by adjusting the amounts of the two components and the extent that the bioactive agent is enclosed within the vesicles. The bioactive agent can be added in the forming of one or bother of components (i) and (ii), or separately.

Also provided is a method of optimizing a delivery composition comprising forming two or more such delivery compositions by
(i) forming a first intermediate lipid component comprising significant amounts of type A lipid, fatty acid, and bilayer-stabilizing steroid(s) and substantially comprising the lipid particles,
(ii) forming a second intermediate lipid component comprising significant amounts of type A lipid, fatty acid, and bilayer-stabilizing steroid(s) and substantially comprising the lipid vesicles, and
(iii) mixing (i) and (ii),
wherein the two or more delivery compositions vary by the lipid composition of (i) or (ii), or by varying the incorporation of bioactive agent into said lipid vesicles;
testing one or more pharmacokinetic parameters of the delivery compositions; and identifying one or a subset of the delivery compositions with more favorable pharmacokinetic parameter(s). Which pharmacokinetic parameter(s) are more favored will vary with the bioactive agent, as will be recognized by those of ordinary skill in the pharmaceutical delivery arts.

### Brief Description of the Drawings

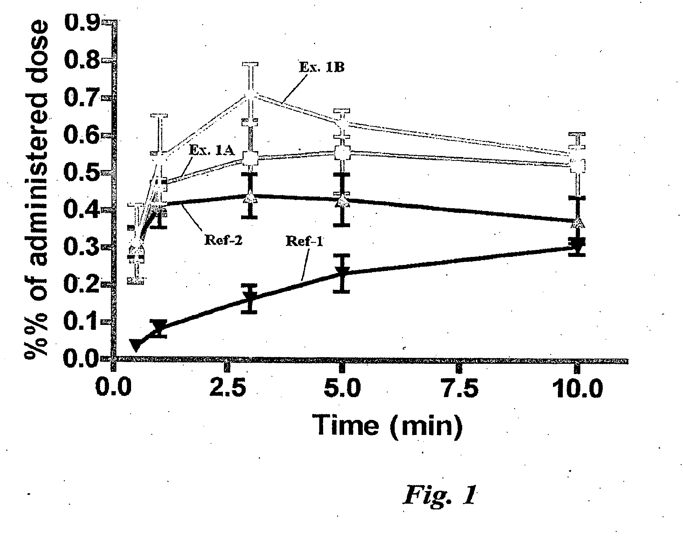

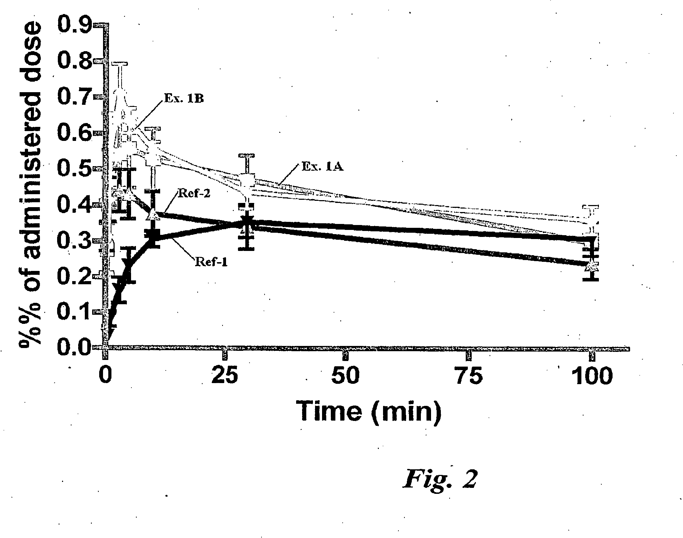

Figures 1 and 2 show time courses for blood levels of testosterone after nasal administration in two compositions according to the invention, and two comparative compositions.

### Definitions

The following terms shall have, for the purposes of this application, the respective meanings set forth below.

### • bioactive agent

A bioactive agent is a substance such as a chemical that can act on a cell, virus, tissue, organ or organism, including but not limited to drugs (i.e., pharmaceuticals) to create a change in the functioning of the cell, virus, organ or organism to achieve a pharmaceutical or therapeutic effect.

### • cell-surface disruptor

A cell surface disruptor is (a) a detergent or (b) an organic solvent; wherein such detergent is (a) a micelle-forming detergent that is stronger than phospholipid, ceramide(s), sphingomyelin(s) or glucocerebroside(s) (in a form typically found in cell membrane) and (b) not a fatty acid or salt thereof that is C8 or higher. A "modified cell-surface disruptor" is not a fatty acid or salt thereof that is C10 or higher.

### • essentially lacking a cell-surface disruptor

A composition is essentially lacking cell-surface disruptors if the amount present is zero or less than the amount that can cause irritation by cell-surface disruption. For example, a cell-surface disruptor might be present due to its use in facilitating the formulation of the composition (such as a carrier for a component that will be substantially diluted), but the amount in the final composition will be of no consequence as a cell-surface disruptor.

### • flexible, soluble polymer

A flexible, soluble polymer is a polymer effective to, when positioned on the outside of a bilayer-enclosed vesicle, to increase the stability of the vesicle.

### • lipid particle

Lipid particles are the result from melting the lipid fraction (described below) in conjunction with mild homogenization and letting it to cool. Lipid particles may thus be relatively heterogeneous, containing for example large or small particles, micro scale lumps, crystals, bilayer fragments and or multilamellar vesicles of different sizes and lamillarity, or the like.

### • lipid-phase anchoring hydrophobic moiety

A lipid-phase anchoring hydrophobic moiety is used as a covalent conjugate with a flexible, soluble polymer. The lipid-phase anchoring hydrophobic moiety associates, for example, with the bilayer of a vesicle with sufficient stability to keep conjugated polymer predominantly anchored to lipid and positioned to increase the stability of the vesicles.

### • mucosal delivery

Mucosal delivery refers to bioactive agent delivery to mucosal tissues, including without limitation nasal, buccal (including gums or cheeks), vaginal, rectal and urethral tissues. Delivery can be to systemic regions via the vasculature of the tissue, or local.

### • significant amounts of type A lipid, fatty acid and a bilayer-stabilizing steroid

A bioactive agent delivery composition has this "significant amount" if a comparable composition were made without the otherwise additional lipids and were nonetheless effective as a delivery vehicle, even if not as effective as the unmodified composition.

### • substantially soluble polymer

A substantially soluble polymer is one that, if it associates to some degree with lipid aggregates, does so less strongly than does the polymer conjugate described above.

### • treatment

"Treating" a disease, disorder or condition includes delaying or ameliorating the progression or initiation of disease, disorders or conditions, including symptoms or complications thereof. Given appropriate bioactive agents, any animal can be treated, including mammals such as humans.

Additional terms are defined in context in the following discussion.

### Detailed Description of the Invention

### Lipid Components

The lipids primarily used to make the two lipid aggregates used in the invention, vesicles and lipid-filled particles, are (i) type A lipids, (ii) fatty acids and (iii) bilayer-stabilizing steroid(s). "Type A" lipid is one or more of any of phospholipid, ceramide(s) sphingomyelin(s), glucocerebroside(s) and conjugate(s) of lipid-phase anchoring hydrophobic moieties and flexible, soluble polymers.

The phospholipid of the type A lipid component can be a mixture of different phospholipid types, including minor amounts of lysophospholipids. In certain embodiments, 5 mole % or more of the phospholipid has a head group with no net charge. For example, the phospholipid can be made up of phosphatidylcholine or phosphatidylethanolamine. In certain embodiments, 10 mole % or more, or, 15 mole % or more, or, 20 mole % or more, or, 25 mole % or more, or, 30 mole % or more, or, 40 mole % or more, 50 mole % or more, 60 mole % or more, 70 mole % or more, 80 mole % or more, 90 mole % or more, of the type A lipid has a head group with no net charge. Typically, only a small percentage, such as 10 mole % or less, of the type A lipid is lysophospholipid. In certain embodiments, 8 mole % or less, or, 7 mole % or less, or, 6 mole % or less, or, 5 mole % or less, or, 4 mole % or less, or, 3 mole % or less, 2 mole % or less, 1 mole % or less, 0.5 mole % or less, is lysophospholipid.

Fatty acyl components of the type A lipids can, for example, be of any composition found in a natural source. Or, the fatty acyl component can be hydrogenated to remove substantially all or a portion of any unsaturation. In this context, substantially all is hydrogenated in the presence of excess hydrogen source to a point where the conversion rate decreases such that additional hydrogenation is only of marginal utility. Hydrogenation can serve to increase the long-term stability of the delivery composition.

In certain embodiments, the fatty acyl component is selected such that 50 mole % or more is C12 or higher, or C14, or C16 or higher. In certain embodiments, the fatty acyl component is selected such that 50 mole % or more is C22 or lower, or C20 or lower, or C18 or lower. In certain embodiments, 75 mole % or more of the fatty acyl component is from C12 or C14 or C16 to C22 or C20 or C18. In certain embodiments, 80 mole % or more, 85 mole % or more, 90 mole % or more, 95 mole % or more, 97 mole % or more, 98 mole % or more, or 99 mole % or more, meets one of the size parameters of this paragraph.

A conjugate of a lipid-phase anchoring hydrophobic moiety and a flexible, soluble polymer can be, for example, a conjugate of a type A lipid and a polymer such as polyethylene glycol. Other hydrophobic materials can be used to anchor the polymer to a lipid or bilayer phase, so long as the association is sufficiently stable. One exemplary conjugate is distearoyl-phosphatidylethanolamine-polyethylene glycol (DSPE-PEG). The conjugated polyethylene glycol can have an average molecular weight of, for example 2000. In certain embodiments, the average molecular weight of the flexible, soluble polymer is 500 or more, 750 or more, or 1000 or more. In certain embodiments, the average molecular weight of the polymer is 5000 or less, 4000 or less, or 3000 or less.

If present, the contribution of the lipid anchor portion of the conjugate to the overall aggregate-forming lipid is typically relatively low, such as 10 mole % or less. In certain embodiments using the conjugate, the contribution is 9 mole % or less, or, 8 mole % or less, or, 7 mole % or less, or, 6 mole % or less, or, 5.5 mole % or less, or, 5 mole % or less. In certain embodiments using the conjugate, the contribution is 1 mole % or more, or, 2 mole % or more, or, 3 mole % or more, or, 4 mole % or more, or, 4.5 mole % or more, or, 5 mole % or more.

Other polymers besides polyethylene glycol can be used, provided sufficient biocompatibility, flexibility and water solubility. Without being bound to theory, it is believed that the polymer stabilizes the lipid aggregates by physically keeping them separate, thereby limiting fusions that change the properties of the lipid aggregates. Other flexible, soluble polymers can include polyvinylpyrrolidone (PVP), polyvinyl alcohol (PVA), monosial gagnlioside, and the like.

Without being bound to theory, it is believed that the conjugate, while stabilizing the lipid aggregates in the composition before use, also help adhere lipid aggregates to the mucosal membrane as the composition spreads along such membrane. This latter function can be substituted, to some degree, with optional non-anchored polymer discussed below.

The fatty acid can, for example, be of any composition found in a natural source, including hydrolysis of esterified fatty acids. Or, the fatty acid component can be hydrogenated to remove substantially all or a portion of any unsaturation. In certain embodiments, the fatty acid component is selected such that 50 mole % or more is C12 or higher, or C 14, or C16 or higher. In certain embodiments, the fatty acid component is selected such that 50 mole % or more is C22 or lower, or C20 or lower, or C18 or lower. In certain embodiments, 75 mole % or more of the fatty acid component is from C12 or C14 or C16 to C22 or C20 or C18. In certain embodiments, 80 mole % or more, 85 mole % or more, 90 mole % or more, 95 mole % or more, 97 mole % or more, 98 mole % or more, or 99 mole % or more, meets one of the size parameters of this paragraph.

The bilayer stabilizing steroid or steroid analog is typically cholesterol, a fatty acyl ester of cholesterol, or an analog thereof, such as ergosterol, cholestanol, 7-dehydrocholesterol, lanosterol, or the like. Any steroid or steroid analog that stabilizes the bilayer of the vesicles can be used, though steroids or analogs with substantial hormone activity are typically avoided unless intended for use as the bioactive agent.

Of the lipids used to make the lipid aggregates, the contribution of type A lipids can be, for example 10 mole % or more, 15 mole % or more, 17.5 mole % or more, 20 mole % or more, 22 mole % or more, 24 mole % or more, 26 mole % or more, 28 mole % or more, or 30 mole % or more. Or, the weight contribution of type A lipids can be, for example 95 mole % or less, 90 mole % or less, 85 mole % or less, 80 mole % or less, 75 mole % or less, 70 mole % or less, 65 mole % or less, 60 mole % or less, or 50 mole % or less. For this purpose, only the weight contribution of the lipid anchoring portion of any conjugate of a lipid-phase anchoring hydrophobic moiety and a flexible, soluble polymer.

Of the lipids used to make the lipid aggregates, the contribution of fatty acids can be, for example 15 mole % or more, 17.5 mole % or more, 20 mole % or more, 22.5 mole % or more, 25.5 mole % or more, 27.5 mole % or more, or 30 mole % or more. Or, the weight contribution of fatty acids can be, for example 60 mole % or less, 55 mole % or less, 52.5 mole % or less, 50 mole % or less, 47.5 mole % or less, 45 mole % or less, 44 mole % or less, 42 mole % or less, or 40 mole % or less.

Of the lipids used to make the lipid aggregates, the contribution of bilayer stabilizing steroid(s) can be, for example 5 mole % or more, 10 mole % or more, 15 mole % or more, 17.5 mole % or more, 20 mole % or more, 21 mole % or more, or 22 mole % or more. Or, the weight contribution of bilayer stabilizing steroid(s) can be, for example 50 mole % or less, 45 mole % or less, 40 mole % or less, 35 mole % or less, 32.5 mole % or less, 30 mole % or less, 28 mole % or less, 27 mole % or less, or 26 mole % or less.

The weight contribution of these lipid components (type A, fatty acid, bilayer stabilizing steroid) to the delivery composition can in general be, for example, 10 % or less, or 8 % less, or 6 % less, or 5 % less, or 4.5 % less, or 5 % less, or 3.5 % less, or 2 % less, or 2.5 % less. The weigh contribution can also be, for example, 0.1 % or more, or 0.2 % more, or 0.5 % more, or 1 % more, or 1.5 % more, or 2 % more. For oral delivery compositions, the weight contribution of these lipid components (type A, fatty acid, bilayer stabilizing steroid) can be, for example, 15 % or less, 12.5 % or less, 10 % or less, or 8 % less, or 6 % less, or 5 % less, or 4.5 % less, or 5 % less, or 3.5 % less, or 2 % less, or 2.5 % less. The weigh contribution can also be, for example, 0.1 % or more, or 0.2 % more, or 0.5 % more, or 1 % more, or 1.5 % more, or 2 % more.

These lipid components are similar to the types of lipids found in biological membranes (Stryer, Biochemistry, Freeman and Company, New York, 1981; Rilfors, Lindblom, Colloid Surface B 26, 11512, 2002, Briigger, Erben et al, Proc. Natl. Acad. Sci USA, 94, 2339, 1997). Without being bound to theory, it is believed that such similarity can increase the speed of healing from any disruptions caused by the delivery composition.

### Other Delivery Composition Components

Among other components for the delivery composition, one can add small molecule solubility enhancers or uptake enhancers, such as glycerol or propylene glycol. The weight contribution of such compounds to the delivery composition can be, for example, 5 % or less, or 4 % less, or 3.5 % less, or 3 % less, or 2.5 % less, or 2 % less. The weigh contribution can also be, for example, 0.1 % or more, or 0.2 % more, or 0.5 % more, or 1 % more, or 1.5 % more, or 2 % more.

Mucosal adhesion-promoting polymers or gums can be included. The weight contribution of such compounds to the delivery composition can be, for example, 5 % or less, or 3 % less, or 2 % less, or 1 % less, or 0.75 % less, or 0.5% less, or 0.3 % less, or 0.2 % less. The weigh contribution can also be, for example, 0.01 % or more, or 0.02 % more, or 0.03 % more, or 0.05 % more, or 0.075 % more, or 0.1 % more.

Buffering agents, salts or other tonicity adjusters, and titrants can also be added. The composition is typically adjusted to be isotonic with the biological fluid typically secreted at the site of application. Buffering agents include phosphate salts, such as mono-sodium phosphate, mono-potassium phosphate, disodium phosphate, dipotassium phosphate, and the like. Salts include sodium chloride. Preservatives can also be added.

In certain embodiments, organic solvents are avoided, or amounts that would be effective as transdermal enhancers are avoided. For this purpose, glycerol and propylene glycol in amounts less than 4 wt % are not such solvents. Such solvents, while enhancing the delivery of bioactive agents, can be damaging to mucosal membranes, and lead to patient discomfort. In certain embodiments, these solvents are found in small amounts, for example, where the bioactive agent is conveniently introduced in solvent including a small amount of such solvents. The weight contribution of such compounds to the delivery composition can be, for example, 10 % or less, or 5 % less, or 3 % less, or 2 % less, or 1 % less, or 0.5 % less, or 0.3 % less, or 0.2 % less, or 0.1 % less.

Detergent activity in an amount to cause irritancy is, in many embodiments, avoided. For example, the compositions are formulated to avoid the irritancy of transmucosal delivery compositions containing such detergents as sodium lauryl sulfate or sodium lauroyl lactylate. Thus, with such embodiments, depletion of mucosal lipids is believed to be limited. Such side effects can be minimized with lipids and excipients that mimic biological membrane and its environment.

In certain embodiments, the delivery composition contains enzyme inhibitors selected to minimize enzymatic degradation of a bioactive agent. Thus, the enzyme inhibitors can be present in amounts that increase the contact time between the bioactive agent and a mucosal tissue. For example, for bioactive agents containing peptide bonds, one or more protease inhibitors may be appropriate. Such inhibitors can be cysteine protease inhibitors, serine protease inhibitors (including serpins), trypsin inhibitors, threonine protease inhibitors, aspartic protease inhibitors, metalloprotease inhibitors, or the like. Similarly, glycosidic bonds, nucleotide bonds, phosphate ester bonds, phosphoamide bonds, and other hydrolytic bonds found in nature can be protected with appropriate enzyme inhibitors.

### Stabilisation of Lipid Aggregates

One form of stabilization against the formation of sediments is the use of the conjugate of anchoring hydrophobic moiety and a flexible, soluble polymer described above. It believed that with the selection of appropriate polymers or polymer mixtures and appropriate higher concentrations, more soluble polymers can also serve this role. To a certain extent, the mucosal adhesion-promoting polymers or gums described above can serve this function, though in some cases higher concentrations are needed. Concentration and selection of polymer can provide an effective coating of the aggregates, which is believed to provide stabilization and enhance delivery of the bioactive agent.

A stabilizing effective amount of such polymers is an amount that increases the stability against sediments of a relevant composition of lipid aggregates adjusted so that lipid is 2.5% by weight, and other composition components are kept at the same concentration.

### Bioactive Agents

Without being bound by theory, it is believed that the delivery system can effectively delivery bioactive agents with a variety of properties, though the mode of delivery enhancement may vary somewhat with the type of bioactive agent. For example:
- With hydrophobic bioactive agents, the lipid aggregates of both types can be expected to carry the active.
- With more water-soluble bioactive agents that nonetheless have hydrophobic or other sticky elements, these can be dissolved within the vesicles of the ultra-fine fraction, adhered to both types of lipid aggregates, and/or dissolved outside of the aggregates.
- With less sticky water-soluble bioactive agents, these can be dissolved within the vesicles, and reside outside the aggregates - with the relative amounts determined by the particular production methods. In this case, the lipid particles are believed to function primarily to slow the clearance of the delivery system from the nasal mucous membranes.

The amount of the bioactive agent in the transmucosal composition will vary with their pharmacological properties, and the form of their association with the lipid aggregates, among other things. Where the bioactive agent is sufficiently hydrophobic that it can be expected to associate with the lipid components of the lipid aggregates, then the effect of the bioactive agent on the properties of the aggregates should be monitored, and the lipid composition adjusted as appropriate.

In certain particular embodiments, 1 mole % of the bioactive agent or more is associated with the vesicles and/or the lipid-filled particles. Or, 2 mole % or more, 5 mole % or more, 10 mole % or more, 15 mole % or more, or 20 mole % or more, or 25 mole % or more, or 30 mole % or more, or 35 mole % or more, or 40 mole % or more, or 45 mole % or more, or 50 mole % or more, is associated with the vesicles and/or the lipid-filled particles.

As outlined above, the delivery composition is anticipated to provide delivery benefits for bioactive agents with a wide variety of physical properties. In certain embodiments, however, the bioactive agent (or agents) is of MW about 1000 or less and has an octanol-water partition coefficient of 1 or higher. Or, the coefficient is 2 or higher, or 5 or higher, or 10 or higher. For example, the bioactive agent (or agents) can be steroid hormones.

In certain embodiments, the bioactive agent (or agents) is a polypeptide. For example, the bioactive agent may be a polypeptide of 2 to 20 amino acid (or amino acid analog) residues. Or, for example, the bioactive agent may be a polypeptide of 21 to 60 amino acid (or amino acid analog) resides. Or, for example, the bioactive agent may be a polypeptide of greater than 60 amino acid (or amino acid analog) residues.

In certain embodiments, the bioactive agent (or agents) have an octanol-water partition coefficient of 1 or less, but have sufficient amphipathic nature that more associates with the aggregates than would a correspondingly formulated composition using a compound of the same or lesser octanol-water partition coefficient and minimal amphipathic nature.

### Lipid Vesicles

The two types of lipid aggregates are typically produced separately, and combined for use. The fraction used to create the vesicles can be termed the "ultra-fine fraction."

Using lipid compositions such as are described herein, bilayer-enclosed vesicles can be made typically with methods that direct sufficient oscillatory energy or other means (e.g. mechanical or thermal) per unit volume - at once or by serially applying such energy to different sub-volumes. Sonicating devices, for example, can be used. Or, appropriate high pressure homogenizers can be used, such as of a Rannie homogenizer from Invensys APV (Fluid Handling & Homogenisers, Lake Mills, WI). The pressure of the homogenizer can be set, for example, from about 10,000 to 40,000 psi, such as 21,756 psi (1500 bar). An example of a sonicator is Soniprep 150, manufactured by Sanyo Gallencamp Plc. Ultrasound radiation is transmitted by high frequency vibrations via a titanium alloy probe from a transducer that converts electrical energy to mechanical energy. The diameter of the probe tip can vary. An example of a diameter of a probe tip is about 9.5 mm. The amplitude at which the sonication can be performed can vary. An example of an amplitude is 10 microns for 30 minutes.

The vesicle formation is typically conducted at a relatively elevated temperature, such as a temperature of 45 °C or more, or 50 °C or more, or 55 °C or more, or 60 °C or more, or 65 °C or more. The temperature can, for example, be 75 °C or less, or 70 °C or less, or 65 °C or less. The bioactive agent(s) may affect the choice of temperature, with the temperature moderated for more labile bioactive agents. The pH obtained from the vesicle formation can be selected in view of the properties of the bioactive agent.

Without being bound to theory, it is believed that the use of smaller vesicles with associated bioactive agent can provide faster initial uptake of the bioactive agent. Thus, depending on the pharmacokinetic profile desired, the amount and size of the vesicles can be varied. Typically, to obtain smaller vesicles, more energy has to be applied to the production process. For example, using the Rannie homogenizer, it may be appropriate to pass the production suspension two or more times through a homogenization cycle. Delays and cooling between the applications of energy can minimize excess heating.

In certain embodiments, the average vesicle size can be, for example, 500 nm or less, or 450 nm less, or 400 nm less, or 350 nm less, or 300 nm less, or 250 nm less, or 200 nm less, or 150 nm less, or 100 nm less. And/or, the average vesicle size can be, for example, 20 nm or more, or 30 nm more, or 40 nm more, or 45 nm more, or 50 nm more, or 75 nm more, or 100 nm more, or 150 nm more, or 200 nm more. Size determination can be by light scattering, using a Malvern Autosizer (Malvern Instruments Ltd., Malvern, Worcestershire, UK), or a device calibrated to give comparable results.

Electron-microscopic analysis shows that the predominate morphology of lipid aggregates is unilamellar vesicles.

### Lipid Particles

The fraction used to create the lipid particles can be termed the "disperse fraction."

The lipid particles can be made by passing aqueous suspensions of the lipid components through dispersing equipment, such as the Dispermix device from Ystral gmbh (Ballrechten-Dottingen, Germany). These particles typically have a wide size distribution, which is typically of sizes larger than found in the ultra-fine fraction, such as from 1000 nm (1 micron). In some embodiments, the upper sizes may be as high as 20 or 30 microns. Average size can be determined by measuring an appropriate sampling by microscope.

Particle formation is typically conducted at a relatively elevated temperature, such as a temperature of 45 °C or more, or 50 °C or more, or 55 °C or more, or 60 °C or more, or 65 °C or more. The temperature can, for example, be 75 °C or less, or 70 °C or less, or 65 °C or less. The bioactive agent(s) may affect the choice of temperature, with the temperature moderated for more labile bioactive agents. The pH obtained from the particle formation can be selected in view of the properties of the bioactive agent.

Without being bound by theory, it is believed that the particles are predominantly surrounded by a lipid monolayer. Lipid components can be selected such that both the ultra-fine fraction and the disperse fraction can be formed from substantially the same lipids.

### Mixing Fractions

The disperse fraction and the ultra-fine fraction can be mixed to form the delivery system. When conducting this mixing, care can be taken to avoid temperatures above a given boundary, such as 35 °C.

The amount of bioactive agent in each of the lipid fractions, and the relative amount of the lipid components of the fractions can be varied as indicated by empirical studies of the resulting pharmacokinetic profile.

### Oral Dosage Forms

For oral dosage forms, for lipid content may be higher, allowing, among other things, for dilution during the course of delivery. The composition is expected to provide facilitate bioactive agent delivery across intestinal epithelium, such as the epithelium of the small intestine (e.g., duodenum, jejunum and ileum) or large intestine (e.g., colon tract).

The delivery composition in one embodiment is provided in a capsule, such as a gelatin capsule. Or, the composition can be encapsulated in multiple smaller particles. The composition of the encapsulating material can be selected in view of the targeted site of delivery, as is known in the art. In another embodiment, the delivery composition is provided as a liquid suspension or dispersion.

In certain embodiments, the delivery composition is dried to allow formulation in dry forms, such as tablets or powder-filled capsules. Drying is typically accomplished by lyophilization. Lyophilization can be done with added protective agents, such as sucrose, raffinose, maltose, lactose, trehalose, or the like. Coatings for delivering such dry powders into various parts of the intestine are known in the art. Coatings for delivery to the colon, for example, are discussed in Bourgeois et al., Am. J. Drug Deliv. 3:171, et seq., 2005.

Where constituted in vesicles or particles prior to lyophilization, it is anticipated that these structures will be reconstituted upon addition of water, such as water from the site of administration. The size of the aggregates, and the distribution of lipid between the two types of aggregates (if both present), may shift somewhat with reconstitution.

### Pulmonary Dosage Forms

For pulmonary delivery, the composition generally does not require modification. It can, however, be useful to control the size of lipid particles to limit the number that are not available to pulmonary tissue due to size. Thus, lengthier homogenization may be applied to limit the lipid particles of size greater than about 5 micron.

In some embodiments, the dried form described in the preceding section is used. The dry material can be spray-dried or micronized to provide an appropriate powder.

Delivery devices for liquid suspensions and dry powder are known. For example, the following pulmonary delivery devices are commercially available: Direct-Haler™ (Direct-Haler A/S, Copenhagen, Denmark), Mystic™ (Ventaira, Columbus, Ohia), Exubera™ (Pfizer, New York, NY), SoloVent™ (BB Technologies, Franklin Lakes, NJ).

### Mucosal Spraying

Any spray device, such as those used for Afrin nasal sprays, can be used to deliver bioactive agent to mucosal tissue. As will be recognized by those of skill in the art, more than one source vessel can be used to hold the composition, or parts thereof, prior to spraying. Mixing structures can be incorporated into the plumbing in which streams from two source vessels are joined.

### Example 1A

An ultra-fine fraction was made using the following ingredients:

| **Component** | **Amount (wt %)** | **Gram wt to 100g** |
|---|---|---|
| Water | 93.0 % | 93.0 |
| Phospholipon 90H | 1.1% | 1.1 |
| Palmitic acid | 0.7% | 0.7 |
| Cholesterol | 0.7% | 0.7 |
| K₂HPO₄ | 0.43% | 0.43 |
| KH₂PO₄ | 0.34% | 0.34 |
| Phenonip | 0.25% | 0.25 |
| Xanthan gum | 0.1% | 0.1 |
| Testosterone | 0.025% | 0.025 |
| Propylene glycol | 1% | 1.0 |
| Glycerol | 1% | 1.0 |
| NaCl | 0.9% | 0.9 |
| DSPE-PEG2000 | 0.4% | 0.4 |
| 5M NaOH | 0.1% | 0.1g=1.0g 0.5M |

The formulation is designed to provide an approximately neutral pH. The vesicles are formed with a Rannie homogenizer operated at 1500 bar for two passes through the homogenizer. The temperature of the forming liquid is kept at approximately 70 °C during homogenization, then allowed to cool to room temperature.

A disperse fraction was made using the following ingredients:

| **Component** | | **Amount** (wt %) | **Gram wt to 100g** |
|---|---|---|---|
| Water | | 93.0% | 93.0 |
| Phospholipon 90H | | 1.1% | 1.1 |
| Palmitic acid | | 0.7% | 0.7 |
| Cholesterol | | 0.7% | 0.7 |
| (a) | K₂HPO₄ | 0.43% | 0.43 |
| (b) | KH₂PO₄ | 0.34% | 0.34 |
| (c) | Phenonip | 0.25% | 0.25 |
| Xanthan gum | | 0.1% | 0.1 |
| Testosterone | | 0.025% | 0.025 |
| Propylene glycol | | 1% | 1.0 |
| Glycerol | | 1% | 1.0 |
| DSPE-PEG2000 | | 0.4% | 0.4 |
| NaCl | | 0.9% | 0.9 |
| 5M NaOH | | 0.1% | 0.1g=1.0g 0.5M |

The formulation is designed to provide an approximately neutral pH. The particles are formed with a Dispermix dispersing device operated for three minutes. The temperature of the forming liquid is kept at approximately 70 °C during homogenization, then allowed to cool to room temperature.

The two fractions are mixed 1:1 (wt) by gentle stirring, taking care to avoid temperatures in excess of 35 °C.

### Example 1B

The same fractions described in Example 1A are made, except that the amount of testosterone in the ultra fine fraction is doubled, and testosterone is omitted from the disperse fraction.

### Example 2

Each Rat is anesthetized with isoflurane or propofol and positioned on a thermostated heating pad. A catheter is put into a tail vein for administration of 20 IE heparin. Arteria femoralis is catheterized for blood sampling. A closed catheter is inserted into the oesophagus to the posterior part of the nasal cavity. The nasopalatine passage is closed with an adhesive agent to prevent drainage of the nasally administered test solution. The test substance is deposited nasally in a volume of 30-100 µl. Blood samples are taken starting shortly after drug administration. Blood sampling is performed at intervals covering 180 minutes. Each blood sample is 0.5 ml and totally 10 % of the blood volume is taken. After the end of the study the animals are euthanized with an i.v. injection of pentobarbital. The blood samples are analyzed for content of the radioactive drug.

Using testosterone with a radioisotope label, blood levels of testosterone are found as illustrated in Figures 1 and 2. The data are obtained from 7-8 rats per treatment. The administered compositions are those of Examples 1A and 1B, and 1 % Polysorbate 80 in a saline solution containing 0.025 % wt/wt testosterone (Ref-1) and 0.025 % wt/wt testosterone in rape seed oil (Ref-2).

## Claims

1. A delivery composition comprising:
an aqueous carrier;
a lipid component suspended in the aqueous carrier, comprising significant amounts of type A lipid, fatty acid, and bilayer-stabilizing steroid(s),
wherein type A lipid is one or more of any of phospholipid, ceramide(s), sphingomyelin(s), glucocerebroside(s) or a conjugate of phospholipid, ceramide(s),
sphingomyelin(s) or glucocerebroside(s) and flexible soluble polymers, wherein the contribution of the phospholipid, ceramide(s), sphingomyelin(s) or
glucocerebroside(s) portion of the conjugate to the lipid component is 10 mole % or
less,
the lipid component formulated in the aqueous carrier to have:
a) a lipid particle component comprising particles of said lipids and having an average diameter of 1 micron or more, said particles being surrounded by a lipid monolayer, and
b) a vesicle component comprising vesicles enclosed by a single lipid bilayer, the bilayer comprising said lipids; and
a bioactive agent suitable for transmucosal systemic delivery;
wherein the delivery composition is packaged with a label with directions for mucosal, pulmonary or oral administration.

2. A delivery composition comprising:
an aqueous carrier;
a lipid component suspended in the aqueous carrier, comprising significant amounts of type A lipid, fatty acid, and bilayer-stabilizing steroid(s),
wherein type A lipid is one or more of any of phospholipid, ceramide(s), sphingomyelin(s), glucocerebroside(s), or a conjugate of phospholipid, ceramide(s), sphingomyelin(s) or glucocerebroside(s) and flexible soluble polymers, wherein the contribution of the phospholipid, ceramide(s), sphingomyelin(s) or
glucocerebroside(s) portion of the conjugate to the lipid component is 10 mole % or less,
the lipid component formulated in the aqueous carrier to have:
a) a lipid particle component comprising particles of said lipids and having an average diameter of 1 micron or more, said particles being surrounded by a lipid monolayer, and
b) a vesicle component comprising vesicles enclosed by a single lipid bilayer, the bilayer comprising said lipids; and
a bioactive agent suitable for transmucosal systemic delivery;
for use in mucosal, pulmonary or oral administration of the bioactive agent to a patient.

3. The delivery composition of claim 1 or 2, wherein the composition is essentially lacking a cell-surface disruptor.

4. The delivery composition of any one of claims 1 to 3, wherein the bioactive agent is sufficiently hydrophobic to associate with the lipid component and/or the bioactive agent is a polypeptide.

5. The delivery composition of any one of claims 1 to 4, wherein the vesicles of the vesicle component have average diameter of 500 nm or less.

6. The delivery composition of any one of claims 1 to 5, wherein the lipid particle component is present in an amount effective to increase retention of the vesicle component at a mucosal surface.

7. The delivery composition of any one of claims 1 to 6, wherein the type A lipid comprises the conjugate of phospholipid, ceramide(s), sphingomyelin(s) or glucocerebroside(s) and flexible soluble polymers.

8. The delivery composition of anyone of claims 1 to 7, wherein the contribution of the type A lipid to the lipid aggregates is 15 mole % or more.

9. The delivery composition of any one of claims 1 to 8, wherein the composition is made by
(i) forming a first intermediate lipid component comprising significant amounts of type A lipid, fatty acid, and bilayer-stabilizing steroid(s),
wherein type A lipid is one or more of any of phospholipid, ceramide(s), sphingomyelin(s), glucocerebroside(s), or a conjugate of phospholipid, ceramide(s), sphingomyelin(s) or glucocerebroside(s) and flexible soluble polymers, wherein the contribution of the phospholipid, ceramide(s), sphingomyelin(s) or glucocerebroside(s) portion of the conjugate to the lipid component is 10 mole % or less,
the lipid component formulated in the aqueous carrier to have:
a) a lipid particle component comprising particles of said lipids and having an average diameter of 1 micron or more, said particles being surrounded by a lipid monolayer, and
b) a vesicle component comprising vesicles enclosed by a single lipid bilayer, the bilayer comprising said lipids; and
wherein the first intermediate lipid component substantially comprises lipid particles;
(ii) forming a second intermediate lipid component comprising significant amounts of type A lipid, fatty acid, and bilayer-stabilizing steroid(s),
wherein type A lipid is one or more of any of phospholipid, ceramide(s), sphingomyelin(s), glucocerebroside(s), or a conjugate of phospholipid, ceramide(s), sphingomyelin(s) or glucocerebroside(s) and flexible soluble polymers, wherein the contribution of the phospholipid, ceramide(s), sphingomyelin(s) or glucocerebroside(s) portion of the conjugate to the lipid component is 10 mole % or less,
the lipid component formulated in the aqueous carrier to have:
a) a lipid particle component comprising particles of said lipids and having an average diameter of 1 micron or more, said particles being surrounded by a lipid monolayer, and
b) a vesicle component comprising vesicles enclosed by a single lipid bilayer, the bilayer comprising said lipids; and
wherein the second intermediate lipid component substantially comprises lipid vesicles; and
(iii) mixing (i) and (ii).

10. A delivery device for a transmucosal composition comprising:
liquid vessel(s) containing the delivery composition of one of claims 1 to 7; and
a sprayer situated to accept and spray delivery composition from the vessel.

11. The delivery composition of any one of claims 1 to 9 for use in treating a disease, disorder or condition wherein the composition is formulated for administration to a mucosal, lung or intestinal surface of a subject in need of the bioactive agent.

12. The delivery composition of claim 11, wherein the composition is for delivery to a nasal mucosal surface and/or a buccal mucosal surface.

13. The delivery composition of claim 11, wherein the composition is for delivery to the lung.

14. The delivery composition of claim 11, wherein the composition is for oral delivery.

15. The delivery composition of any one of claims 1 to 9, wherein the composition is formed by lyophilization to remove water and wherein the delivery composition has been formulated for oral delivery by compression, encapsulation, or coating.

16. The delivery composition of any one of claims 1 to 9 or 11 to 15, or the delivery device of claim 10, wherein the lipid component comprises 10 mol % or more type A lipid.

17. The delivery composition of any one of claims 1 to 9 or 11 to 15, or the delivery device of claim 10, wherein the lipid component comprises 15 mol % or more fatty acid.

18. The delivery composition of any one of claims 1 to 9 or 11 to 15, or the delivery device of claim 10, wherein the lipid component comprises 5 mol % or more of bilayer-stabilizing steroid(s).

## Patentansprüche

1. Ein Zusammensetzung zur Zufuhr umfassend:
einen wässrigen Träger;
eine im wässrigen Träger aufgelöste Lipidkomponente, umfassend signifikante Mengen von Typ A Lipid, Fettsäure und Doppelschicht-stabilisierendes/n Steroid(en),
wobei das Typ A Lipid ein oder mehr von irgendeinem aus Phospholipid, Ceramid(en), Sphingomyelin(en), Glucocerebrosid(en), oder einem Konjugat von Phospholipid, Ceramid(en), Sphingomyelin(en) oder Glucocerebrosid(en) und flexiblen löslichen Polymeren ist, wobei der Beitrag des Phospholipid-, Ceramid-, Sphingomyelin- oder Glucocerebrosidanteils des Konjugats zur Lipidkomponente 10 Mol-% oder weniger ist,
wobei die im wässrigen Träger formulierte Lipidkomponente zu haben hat:
(a) eine Lipidpartikelkomponente umfassend Partikel jenes Lipids und mit einem durchschnittlichen Durchmesser von 1 Mikron oder mehr, wobei jene Partikel von einer Lipideinzelschicht umgeben sind, und
(b) eine Vesikelkomponente umfassend von einer einzelnen Lipiddoppelschicht eingeschlossene Vesikel, wobei die Doppelschicht jene Lipide umfasst; und
ein bioaktives Agens geeignet zur transmukosalen systemischen Zufuhr;
wobei die Zusammensetzung zur Zufuhr mit einer Kennzeichnung mit Anweisungen für die mukosale, pulmonale oder orale Verabreichung verpackt ist.

2. Ein Zusammensetzung zur Zufuhr umfassend:
einen wässrigen Träger;
eine im wässrigen Träger aufgelöste Lipidkomponente, umfassend signifikante Mengen von Typ A Lipid, Fettsäure und Doppelschicht-stabilisierendes/n Steroid(en),
wobei das Typ A Lipid ein oder mehr von irgendeinem aus Phospholipid, Ceramid(en), Sphingomyelin(en), Glucocerebrosid(en), oder einem Konjugat von Phospholipid, Ceramid(en), Sphingomyelin(en) oder Glucocerebrosid(en) und flexiblen löslichen Polymeren ist, wobei der Beitrag des Phospholipid-, Ceramid-, Sphingomyelin- oder Glucocerebrosidanteils des Konjugats zur Lipidkomponente 10 Mol-% oder weniger ist,
wobei die im wässrigen Träger formulierte Lipidkomponente zu haben hat:
(a) eine Lipidpartikelkomponente umfassend Partikel jenes Lipids und mit einem durchschnittlichen Durchmesser von 1 Mikron oder mehr, wobei jene Partikel von einer Lipideinzelschicht umgeben sind, und
(b) eine Vesikelkomponente umfassend von einer einzelnen Lipiddoppelschicht eingeschlossene Vesikel, wobei die Doppelschicht jene Lipide umfasst; und
ein bioaktives Agens geeignet zur transmukosalen systemischen Zufuhr;
zur Verwendung in der mukosalen, pulmonalen oder oralen Verabreichung des bioaktiven Agens an einen Patienten.

3. Die Zusammensetzung zur Zufuhr von Anspruch 1 oder 2, wobei der Zusammensetzung im Wesentlichen ein Zelloberflächen-Disruptor fehlt.

4. Die Zusammensetzung zur Zufuhr von irgendeinem der Ansprüche 1 bis 3, wobei das bioaktive Agens hinreichend hydrophob ist, um mit der Lipidkomponente zu assoziieren und/oder das bioaktive Agens ein Polypeptid ist.

5. Die Zusammensetzung zur Zufuhr von irgendeinem der Ansprüche 1 bis 4, wobei die Vesikel der Vesikelkomponente einen durchschnittlichen Durchmesser von 500nm oder weniger haben.

6. Die Zusammensetzung zur Zufuhr von irgendeinem der Ansprüche 1 bis 5, wobei die Lipidpartikelkomponente in einer Menge vorhanden ist, um die Retention der Vesikelkomponente an einer mukosalen Oberfläche wirksam zu erhöhen.

7. Die Zusammensetzung zur Zufuhr von irgendeinem der Ansprüche 1 bis 6, wobei das Typ A Lipid das Konjugat von Phospholipid, Ceramid(en), Sphingomyelin(en) oder Glucocerebrosid(en) und flexiblen löslichen Polymeren umfasst.

8. Die Zusammensetzung zur Zufuhr von irgendeinem der Ansprüche 1 bis 7, wobei der Beitrag des Typ A Lipids zum Lipidaggregat 15 Mol-% oder mehr ist.

9. Die Zusammensetzung zur Zufuhr von irgendeinem der Ansprüche 1 bis 8, wobei die Zusammensetzung hergestellt wird durch
(i) Bilden einer ersten intermediären Lipidkomponente umfassend signifikante Mengen von Typ A Lipid, Fettsäure und Doppelschicht-stabilisierendes/n Steroid(en), wobei das Typ A Lipid ein oder mehr von irgendeinem aus Phospholipid, Ceramid(en), Sphingomyelin(en), Glucocerebrosid(en), oder einem Konjugat von Phospholipid, Ceramid(en), Sphingomyelin(en) oder Glucocerebrosid(en) und flexiblen löslichen Polymeren ist, wobei der Beitrag des Phospholipid-, Ceramid-, Sphingomyelin- oder Glucocerebrosidanteils des Konjugats zur Lipidkomponente 10 Mol-% oder weniger ist,
wobei die im wässrigen Träger formulierte Lipidkomponente zu haben hat:
(a) eine Lipidpartikelkomponente umfassend Partikel jenes Lipids und mit einem durchschnittlichen Durchmesser von 1 Mikron oder mehr, wobei jene Partikel von einer Lipideinzelschicht umgeben sind, und
(b) eine Vesikelkomponente umfassend von einer einzelnen Lipiddoppelschicht eingeschlossene Vesikel, wobei die Doppelschicht jene Lipide umfasst; und
wobei die erste intermediäre Lipidkomponente im Wesentlichen Lipidpartikel umfasst;
(ii) Bilden einer zweiten intermediären Lipidkomponente umfassend signifikante Mengen von Typ A Lipid, Fettsäure und Doppelschicht-stabilisierendes/n Steroid(en), wobei das Typ A Lipid ein oder mehr von irgendeinem aus Phospholipid, Ceramid(en), Sphingomyelin(en), Glucocerebrosid(en), oder einem Konjugat von Phospholipid, Ceramid(en), Sphingomyelin(en) oder Glucocerebrosid(en) und flexiblen löslichen Polymeren ist, wobei der Beitrag des Phospholipid-, Ceramid-, Sphingomyelin- oder Glucocerebrosidanteils des Konjugats zur Lipidkomponente 10 Mol-% oder weniger ist,
wobei die im wässrigen Träger formulierte Lipidkomponente zu haben hat:
(a) eine Lipidpartikelkomponente umfassend Partikel jenes Lipids und mit einem durchschnittlichen Durchmesser von 1 Mikron oder mehr, wobei jene Partikel von einer Lipideinzelschicht umgeben sind, und
(b) eine Vesikelkomponente umfassend von einer einzelnen Lipiddoppelschicht eingeschlossene Vesikel, wobei die Doppelschicht jene Lipide umfasst; und
wobei die zweite intermediäre Lipidkomponente im Wesentlichen Lipidvesikel umfasst; und
(iii) Mischen von (i) und (ii).

10. Eine Vorrichtung zur Zufuhr für eine transmukosale Zusammensetzung umfassend:
Flüssigkeitsgefäß(e), die die Zusammensetzung zur Zufuhr von einem der Ansprüche 1 bis 7 beinhaltet/en; und eine Sprühvorrichtung, die so angebracht ist, um die Zusammensetzung zur Zufuhr aus dem Gefäß aufzunehmen und zu versprühen.

11. Die Zusammensetzung zur Zufuhr von einem der Ansprüche 1 bis 9 zur Verwendung in der Behandlung eines Leidens, Störung oder Erkrankung, wobei die Zusammensetzung zur Verabreichung an eine Schleimhaut-, Lungen-, oder Darmoberfläche eines Subjekts, die das bioaktive Agens benötigt, formuliert ist.

12. Die Zusammensetzung zur Zufuhr von Anspruch 11, wobei die Zusammensetzung für die Zufuhr an eine Nasenschleimhautoberfläche und/oder Wangenschleimhautoberfläche ist.

13. Die Zusammensetzung zur Zufuhr von Anspruch 11, wobei die Zusammensetzung zur Zufuhr an die Lunge ist.

14. Die Zusammensetzung zur Zufuhr von Anspruch 11, wobei die Zusammensetzung zur oralen Zufuhr ist.

15. Die Zusammensetzung zur Zufuhr von irgendeinem der Ansprüche 1 bis 9, wobei die Zusammensetzung durch Gefriertrocknen zur Entfernung von Wasser gebildet wird und wobei die Zusammensetzung zur Zufuhr zur oralen Zufuhr durch Zusammendrücken, Verkapselung oder Beschichtung formuliert wurde.

16. Die Zusammensetzung zur Zufuhr von irgendeinem der Ansprüche 1 bis 9 oder 11 bis 15, oder die Vorrichtung zur Zufuhr von Anspruch 10, wobei die Lipidkomponente 10 Mol-% oder mehr Typ A Lipid umfasst.

17. Die Zusammensetzung zur Zufuhr von irgendeinem der Ansprüche 1 bis 9 oder 11 bis 15, oder die Vorrichtung zur Zufuhr von Anspruch 10, wobei die Lipidkomponente 15 Mol-% oder mehr Fettsäure umfasst.

18. Die Zusammensetzung zur Zufuhr von irgendeinem der Ansprüche 1 bis 9 oder 11 bis 15, oder die Vorrichtung zur Zufuhr von Anspruch 10, wobei die Lipidkomponente 5 Mol-% oder mehr Doppelschicht-stabilisierende(s) Steroid(e) umfasst.

## Revendications

1. Composition d'administration comprenant :
- un support aqueux ;
- un composant lipidique en suspension dans le support aqueux, comprenant des quantités significatives de lipide de type A, d'acide gras et d'un ou de stéroïdes de stabilisation de bicouche,
- le lipide de type A étant un ou plusieurs de l'un quelconque de phospholipide, céramide(s), sphingomyéline(s), glucocérébroside(s) ou un conjugué de phospholipide, céramide(s), sphingomyéline(s) ou glucocérébroside(s) et de polymères solubles flexibles, la contribution de la fraction phospholipide, céramide(s), sphingomyéline(s) ou glucocérébroside(s) du conjugué au composant lipidique étant de 10 % en moles ou moins,
- le composant lipidique étant formulé dans le support aqueux pour avoir :
a) un composant particules lipidiques comprenant des particules desdits lipides et ayant un diamètre moyen de 1 micron ou plus, lesdites particules étant entourées par une monocouche lipidique, et
b) un composant vésicules comprenant des vésicules enfermées par une bicouche lipidique unique, la bicouche comprenant lesdits lipides ; et
- un agent bioactif approprié pour une administration systémique transmucosale ;
la composition d'administration étant conditionnée avec une étiquette ayant des indications pour une administration mucosale, pulmonaire ou orale.

2. Composition d'administration comprenant :
- un support aqueux ;
- un composant lipidique en suspension dans le support aqueux, comprenant des quantités significatives de lipide de type A, d'acide gras et d'un ou de stéroïdes de stabilisation de bicouche, le lipide de type A étant un ou plusieurs de l'un quelconque de phospholipide, céramide(s), sphingomyéline(s), glucocérébroside(s) ou un conjugué de phospholipide, céramide(s), sphingomyéline(s) ou glucocérébroside(s) et de polymères solubles flexibles, la contribution de la fraction phospholipide, céramide(s), sphingomyéline(s) ou glucocérébroside(s) du conjugué au composant lipidique étant de 10 % en moles ou moins,
- le composant lipidique étant formulé dans le support aqueux pour avoir :
a) un composant particules lipidiques comprenant des particules desdits lipides et ayant un diamètre moyen de 1 micron ou plus, lesdites particules étant entourées par une monocouche lipidique, et
b) un composant vésicules comprenant des vésicules enfermées par une bicouche lipidique unique, la bicouche comprenant lesdits lipides ; et
- un agent bioactif approprié pour une administration systémique transmucosale ;
en vue d'une utilisation dans l'administration mucosale, pulmonaire ou orale de l'agent bioactif à un patient.

3. Composition d'administration selon l'une des revendications 1 ou 2, dans laquelle la composition n'a sensiblement pas de désintégrateur de surfaces cellulaires.

4. Composition d'administration selon l'une quelconque des revendications 1 à 3, dans laquelle l'agent bioactif est suffisamment hydrophobe pour s'associer avec le composant lipidique et/ou l'agent bioactif est un polypeptide.

5. Composition d'administration selon l'une quelconque des revendications 1 à 4, dans laquelle les vésicules du composant vésicules ont un diamètre moyen de 500 nm ou moins.

6. Composition d'administration selon l'une quelconque des revendications 1 à 5, dans laquelle le composant particules lipidiques est présent dans une quantité efficace pour augmenter la rétention du composant vésicules à une surface mucosale.

7. Composition d'administration selon l'une quelconque des revendications 1 à 6, dans laquelle le lipide de type A comprend le conjugué de phospholipide, céramide(s), sphingomyéline(s) ou glucocérébroside(s) et de polymères solubles flexibles.

8. Composition d'administration selon l'une quelconque des revendications 1 à 7, dans laquelle la contribution du lipide de type A aux agrégats lipidiques est de 15 % en moles ou plus.

9. Composition d'administration selon l'une quelconque des revendications 1 à 8, dans laquelle la composition est obtenue par :
(i) formation d'un premier composant lipidique intermédiaire comprenant des quantités significatives de lipide de type A, d'acide gras et d'un ou de stéroïdes de stabilisation de bicouche, le lipide de type A étant un ou plusieurs de l'un quelconque de phospholipide, céramide(s), sphingomyéline(s), glucocérébroside(s) ou un conjugué de phospholipide, céramide(s), sphingomyéline(s) ou glucocérébroside(s) et de polymères solubles flexibles, la contribution de la fraction phospholipide, céramide(s), sphingomyéline(s) ou glucocérébroside(s) du conjugué au composant lipidique étant de 10 % en moles ou moins,
le composant lipidique étant formulé dans le support aqueux pour avoir :
a) un composant particules lipidiques comprenant des particules desdits lipides et ayant un diamètre moyen de 1 micron ou plus, lesdites particules étant entourées par une monocouche lipidique, et
b) un composant vésicules comprenant des vésicules enfermées par une bicouche lipidique unique, la bicouche comprenant lesdits lipides ; et
le premier composant lipidique intermédiaire comprenant sensiblement des particules lipidiques ;
(ii) formation d'un second composant lipidique intermédiaire comprenant des quantités significatives de lipide de type A, d'acide gras, et d'un ou de stéroïdes de stabilisation de bicouche, le lipide de type A étant un ou plusieurs de l'un quelconque de phospholipide, céramide(s), sphingomyéline(s), glucocérébroside(s), ou un conjugué de phospholipide, céramide(s), sphingomyéline(s) ou glucocérébroside(s) et de polymères solubles flexibles, la contribution de la fraction phospholipide, céramide(s), sphingomyéline(s) ou glucocérébroside(s) du conjugué au composant lipidique étant de 10 % en moles ou moins,
le composant lipidique étant formulé dans le support aqueux pour avoir :
a) un composant particules lipidiques comprenant des particules desdits lipides et ayant un diamètre moyen de 1 micron ou plus, lesdites particules étant entourées par une monocouche lipidique, et
b) un composant vésicules comprenant des vésicules enfermées par une bicouche lipidique unique, la bicouche comprenant lesdits lipides ; et
le second composant lipidique intermédiaire comprenant sensiblement des vésicules lipidiques ; et
(iii) mélange de (i) et (ii).

10. Dispositif d'administration pour une composition transmucosale comprenant :
- un ou des récipients de liquide contenant la composition d'administration selon l'une quelconque des revendications 1 à 7 ; et
- un pulvérisateur positionné pour accepter et pulvériser la composition d'administration à partir du récipient.

11. Composition d'administration selon l'une quelconque des revendications 1 à 9 en vue d'une utilisation dans le traitement d'une maladie, d'un trouble ou d'un état dans lequel la composition est formulée pour une administration à une surface mucosale, de poumon ou intestinale d'un sujet ayant besoin de l'agent bioactif.

12. Composition d'administration selon la revendication 11, dans laquelle la composition est destinée à une administration à une surface mucosale nasale et/ou une surface mucosale buccale.

13. Composition d'administration selon la revendication 11, dans laquelle la composition est destinée à une administration au poumon.

14. Composition d'administration selon la revendication 11, dans laquelle la composition est destinée à une administration orale.

15. Composition d'administration selon l'une quelconque des revendications 1 à 9, dans laquelle la composition est formée par lyophilisation pour éliminer l'eau et dans laquelle la composition d'administration a été formulée pour une administration orale par compression, encapsulation ou enrobage.

16. Composition d'administration selon l'une quelconque des revendications 1 à 9 ou 11 à 15, ou dispositif d'administration selon la revendication 10, dans laquelle/lequel le composant lipidique comprend 10 % en moles ou plus de lipide de type A.

17. Composition d'administration selon l'une quelconque des revendications 1 à 9 ou 11 à 15, ou dispositif d'administration selon la revendication 10, dans laquelle/lequel le composant lipidique comprend 15 % en moles ou plus d'acide gras.

18. Composition d'administration selon l'une quelconque des revendications 1 à 9 ou 11 à 15, ou dispositif d'administration selon la revendication 10, dans laquelle/lequel le composant lipidique comprend 5 % en moles ou plus d'un ou de stéroïdes de stabilisation de bicouche.
